# EUROPEAN PATENT APPLICATION

(11) **EP 4 306 634 A1**
(43) Date of publication of application: **17.01.2024**
(21) Application number: 22767170.8
(22) Date of filing: 08.03.2022
(51) Int. Cl.: C12N 5/0735, C12N 5/10, C12N 15/09, A61K 35/30

(54) **METHOD FOR PRODUCING HYPOIMMUNOGENIC RETINAL PIGMENT EPITHELIAL CELLS**

(30) Priority: 09.03.2021 US 202163158661 P
(71) Applicant: RIKEN, Wako-shi, Saitama 351-0198 (JP)
(72) Inventor: SUGITA, Sunao, Wako-shi, Saitama 351-0198 (JP); TAKAHASHI, Masayo, Wako-shi, Saitama 351-0198 (JP); MASUDA, Tomohiro, Wako-shi, Saitama 351-0198 (JP)
(74) Representative: Weickmann & Weickmann PartmbB
(86) International application number: PCT/JP2022/010124
(87) International publication number: WO 2022/191216

(57) **Abstract**

The present invention provides low immunogenic retinal pigment epithelial cells and methods of producing the cells. The present invention contains a step of obtaining a pluripotent stem cell that does not express major histocompatibility complex (MHC) class II protein or lacks the function thereof (step 1), and a step of inducing a retinal pigment epithelial cell from the pluripotent stem cell obtained in step 1 (step 2).

## Description

### [Technical Field]

The present invention relates to a method for producing retinal pigment epithelial cells with reduced antigenicity (low immunogenic), a production method thereof, and the like.

### [Background Art]

Cell surface proteins called HLA or MHC, which play the most important role in distinguishing between autologous and non-autologous cells, are known. HLA is classified into class I and class II, and class I HLA proteins are expressed in most cell types in the body, whereas class II HLA proteins are expressed only in specific cells.

In recent years, plural attempts have been reported to reduce immune rejection during allogeneic transplantation by modulating HLA protein expression by methods of, for example, knocking out the β2 microglobulin (B2M) gene to eliminate class I and knocking out the regulatory factor X-associated ankyrin-containing protein (RFXANK) gene to eliminate class II, using genome editing technique and the like.

Patent Literature 1 describes cells in which the B2M gene, which is necessary for the cell surface presentation of class I HLA proteins, has been deleted. Since these cells lack the B2M gene, class I HLA protein is not presented on the cell surface. It is therefore considered that the immune reaction during allogeneic transplantation is suppressed. However, when HLA protein is not presented on the cell surface, the cell loses its ability to present antigens. When cells lose the ability to present antigens and are infected with viruses, etc. or become tumors, they can no longer present antigens derived from viruses or tumors, and may consequently assist the growth of viruses and tumors. Also, cells that do not have HLA protein presented on the cell surface are attacked by NK cells that recognize "missing self".

On the other hand, Patent Literature 2 describes iPS cells that retain only HLA-C and have completely disrupted HLA-A and HLA-B genes. It is described that the blood cells obtained by differentiation of this iPS cell can avoid the attack of killer T cells in the same way as the cells lacking the B2M gene in Patent Literature 1, and further that they are relatively less susceptible to the attack of NK cells than the cells of Patent Literature 1.

Furthermore, Patent Literature 3 describes cells deficient in the B2M gene required for cell surface presentation of class I HLA proteins and the RFXANK gene associated with the expression of class II HLA proteins.

Deletion of HLA (MHC) protein which plays an important role in distinguishing between autologous and non-autologous cells can be dangerous. For example, systemic deficiency of MHC class II (MHC class II deficiency) impairs antigen presentation and thus prevents induction of acquired immunity, resulting in humoral/cellular immunodeficiency. On the other hand, deficiency of MHC class I (MHC class I deficiency) causes insufficient removal of the virus and continuous production of IL-8 at the infection site, resulting in cytotoxicity by neutrophils. In the case of nerves, HLA (MHC) proteins are known to be functionally and morphologically involved in synaptic removal that ensures accurate binding.

That is, the clinical application of HLA (MHC) protein deficiency may vary depending on the organ, tissue, and treatment concept.

However, the effect of the absence of expression of HLA protein (not presented on the cell surface) on ocular tissues, such as retinal pigment epithelial cells used for allograft treatment for retinal insufficiency, is unknown.

### [Citation List]

### [Patent Literature]

[PTL 1] WO2012/145384
[PTL 2] WO2019/160077
[PTL 3] WO2013/158292

### [Summary of Invention]

### [Technical Problem]

The present invention aims to provide low immunogenic retinal pigment epithelial cells and production methods thereof and the like.

### [Solution to Problem]

The present inventors have conducted intensive studies with respect to the above-mentioned problems and found that retinal pigment epithelial (RPE) cells obtained by differentiation induction of pluripotent stem cells that do not express major histocompatibility complex (MHC) class II proteins less likely cause immune rejection even when allogeneic transplantation is performed. Sufficient suppression of immune response was observed even when class I was preserved, and suppression of immune rejection and long-term engraftment were confirmed, which resulted in the completion of the present invention.

The present invention may include the following inventions.
[1] A method for producing a low immunogenic retinal pigment epithelial cell, comprising
   step 1: a step of obtaining a pluripotent stem cell that does not express major histocompatibility complex (MHC) class II protein or lacks the function thereof, and
   step 2: a step of inducing a retinal pigment epithelial cell from the pluripotent stem cell obtained in step 1.
[2] The method of the above-mentioned [1], wherein the step 1 is performed by destroying or modifying an MHC class II-related gene.
[3] The method of the above-mentioned [2], wherein the gene is destroyed or modified by genome editing.
[4] The method of the above-mentioned [2], wherein the gene is destroyed or modified by a CRISPR-Cas9 method.
[5] The method of any of the above-mentioned [1] to [4], wherein the MHC class II is human leukocyte antigen (HLA) class II.
[6] The method of any of the above-mentioned [2] to [5], wherein the HLA class II-related gene is a gene encoding at least one selected from regulatory factor X-associated ankyrin-containing protein (RFXANK), regulatory factor v (RFX5), regulatory factor X-associated protein (RFXAP), class II transactivator (CIITA), HLA-DPA (α chain), HLA-DPB (β chain), HLA-DQA, HLA-DQB, HLA-DRA, HLA-DRB, HLA-DMA, HLA-DMB, HLA-DOA, and HLA-DOB.
[7] The method of the above-mentioned [6], wherein the HLA class II-related gene is a gene encoding CIITA.
[8] The method of any of the above-mentioned [1] to [7], wherein the pluripotent stem cell is an embryonic stem cell (ES cell), an EG cell (Embryonic germ cell), or an induced pluripotent stem cell (iPS cell).
[9] The method of the above-mentioned [8], wherein the pluripotent stem cell is derived from human.
[10] A low immunogenic retinal pigment epithelial cell that does not express a major histocompatibility complex (MHC) class II protein or lacks the function thereof.
[11] The cell of the above-mentioned [10], which is produced by the method of any of the above-mentioned [1] to [9].
[12] The cell of the above-mentioned [10] or [11], that expresses a major histocompatibility complex (MHC) class I protein, and does not lack the function thereof.
[13] The cell of any of the above-mentioned [10] to [12], wherein a major histocompatibility complex (MHC) class I-related gene is retained intact.
[14] The cell of the above-mentioned [12] or [13], wherein the MHC class I is human leukocyte antigen (HLA) class I.
[15] The cell of the above-mentioned [14], wherein the HLA class I is HLA-A, HLA-B, or HLA-C.
[16] A pluripotent stem cell for inducing a low immunogenic retinal pigment epithelial cell that lacks a major histocompatibility complex (MHC) class II-related gene or lacks the function thereof.
[17] The cell of the above-mentioned [16], wherein the MHC class II is human leukocyte antigen (HLA) class II.
[18] The cell of the above-mentioned [17], wherein the HLA class II-related gene is a gene encoding at least one selected from regulatory factor X-associated ankyrin-containing protein (RFXANK), regulatory factor v (RFX5), regulatory factor X-associated protein (RFXAP), class II transactivator (CIITA), HLA-DPA (α chain), HLA-DPB (β chain), HLA-DQA, HLA-DQB, HLA-DRA, HLA-DRB, HLA-DMA, HLA-DMB, HLA-DOA, and HLA-DOB.
[19] The cell of the above-mentioned [18], wherein the HLA class II-related gene is a gene encoding CIITA.
[20] The cell of any of the above-mentioned [16] to [19], wherein the pluripotent stem cell is an embryonic stem cell (ES cell), an EG cell (Embryonic germ cell), or an induced pluripotent stem cell (iPS cell).
[21] The cell of the above-mentioned [20], which is derived from human.
[22] A transplantation material comprising the retinal pigment epithelial cell of any of the above-mentioned [10] to [15], or a retinal pigment epithelial cell derived from the pluripotent stem cell of any of the above-mentioned [16] to [21].

### [Advantageous Effects of Invention]

RPE cells derived from pluripotent stem cells in which class I was preserved and only class II was selectively destroyed can sufficiently suppress immune responses in allogeneic transplantation. Cells with minimal genetic modification can avoid the risk caused by failure of class I HLA protein to be presented on the cell surface that, when cells are infected with viruses, etc. or become tumors, antigens derived from the viruses or tumors cannot be presented, which may consequently assist the growth of the viruses and tumors. Another advantage of preserving class I is that the graft (RPE cell) attack by natural killer (NK) cell can be avoided. Due to the expression of classical class I (HLA-ABC) and non-classical class I (HLA-E), the attack of NK cell on the graft is considered to be strikingly reduced.

MHC (HLA) class I is considered to be important in the presentation of endogenous antigens (particularly viral antigens infecting the cells) and cancer antigens. For example, the herpes virus has a very high affinity for the retina and is latent in the retina including the RPE, and the RPE presents viral antigens to eliminate the virus. In addition, since iPS transplants may contain cancer antigens in the grafts, they may be responding to cancer immunity in the same way as described above, and it is considered important to preserve these immune responses.

From the above, the advantage of preserving MHC (HLA) class I seems to be significant.

### [Brief Description of Drawings]

[Fig. 1] Fig. 1 shows the expression profiles of MHC class I and MHC class II in monkey iPS- or ES cell-derived RPE (monkey iPS-RPE or monkey ES-RPE).
[Fig. 2] Fig. 2 shows the results of MHC gene polymorphism analysis in monkey iPS-RPE or monkey ES-RPE.
[Fig. 3] Fig. 3 shows the results of fluorescein fluorescence funduscopic imaging (fluorescein angiography: FA) and optical coherence tomography (OCT) 6 months post-operation after allogeneic transplantation (Allografts) of monkey ES-RPE suspension.
[Fig. 4] Fig. 4 shows the results of immunochemical tissue staining (H&E staining) 6 months post-operation after allogeneic transplantation (Allografts) of monkey ES-RPE suspension.
[Fig. 5] Fig. 5 shows the results of immunohistochemistry (IHC) analysis of retina section 6 months post-operation after allogeneic transplantation (Allografts) of monkey ES-RPE suspension. The length of the scale bars is 50 µm.
[Fig. 6] Fig. 6 shows the results of long-term (2 years) follow-up observation after allogeneic transplantation of monkey ES-RPE (CMK6) sheet (Allografts).
[Fig. 7A] Fig. 7A is a diagram showing the procedure of a lymphocyte-graft mixed reaction test (LGIR) using monkey ES-RPE and monkey iPS-RPE.
[Fig. 7B] Fig. 7B shows the results of lymphocyte-graft mixed reaction test (LGIR) using monkey ES-RPE and monkey iPS-RPE.
[Fig. 8] Fig. 8 shows the morphology of RPE (MHC-II KO iPS-RPE/iPSC-RPE) derived from monkey MHC class II knockout iPS cell and the results of MHC expression thereof.
[Fig. 9] Fig. 9 shows the results of IHC analysis when MHC-II KO iPS-RPE (Kaiyan monkey) was transplanted into an animal model. As a control, wild-type (iPS-RPE(WT)) cells (without knocking out MHC class II gene) were used (Sayori monkey).
[Fig. 10] Fig. 10 shows the results of immunochemical tissue staining (H&E staining) 6 months post-operation after allogeneic transplantation (Allografts) of monkey iPS-RPE (WT) or monkey MHC-II KO iPS-RPE suspensions.
[Fig. 11A] Fig. 11A shows the results of IHC analysis of CD3 expression of retina section 6 months post-operation after allogeneic transplantation (Allografts) of monkey iPS-RPE (WT) or monkey MHC-II KO iPS-RPE suspensions.
[Fig. 11B] Fig. 11B shows the results of IHC analysis of Iba1 expression and CD4 expression of retina section 6 months post-operation after allogeneic transplantation (Allografts) of monkey iPS-RPE (WT) or monkey MHC-II KO iPS-RPE suspensions.
[Fig. 12] Fig. 12 shows the results of infiltration of CD3⁺T cells in retina 6 months post-operation after allogeneic transplantation (Allografts) of monkey MHC-II KO iPS-RPE suspension.
[Fig. 13] Fig. 13 is a diagram summarizing the results of IHC analysis when monkey iPS-RPE (WT) or monkey MHC-II KO iPS-RPE was transplanted.
[Fig. 14] Fig. 14 shows the results of LGIR assay using anti-MHC class II inhibitory antibody.
[Fig. 15A] Fig. 15A shows the results of expression of HLA class I and HLA class II in human HLA knockout iPS cell-derived RPE (HLA KO iPS-RPE).
[Fig. 15B] Fig. 15B shows the results of expression of HLA class I and HLA class II in human HLA knockout iPS cell-derived RPE (HLA KO iPS-RPE) .
[Fig. 16] Fig. 16 shows the results of confirmation of low immunogenicity of human HLA KO iPS-RPE by LGIR assay.
[Fig. 17] Fig. 17 shows the results of confirmation of low immunogenicity of human HLA class II knockout iPS cell-derived RPE (HLA class II KO iPSC-RPE) by LGIR assay.
[Fig. 18] Fig. 18 shows the protocol of CIITA genome editing of monkey iPS cells.
[Fig. 19] Fig. 19 shows the observation results, by color funduscopic photograph (a), fluorescein angiography (FA) (b), fundus autofluorescence (FAF) (c), optical coherence tomography (OCT) (d), H&E staining (e), and IHC analysis (f, g), of grafts 6 months post-operation after allogeneic transplantation of monkey MHC class II KO iPS-RPE cells.
[Fig. 20] Fig. 20 is a diagram confirming HLA class II deficiency by flow cytometry analysis of RPE cells derived from the produced human HLA class II-deficient iPS cells.
[Fig. 21] Fig. 21 shows the results of lymphocyte-graft mixed reaction test (LGIR) using RPE cells derived from human HLA class II-deficient iPS cells.

### [Description of Embodiments]

The embodiments of the present invention are described in detail below.

In the present invention, "low immunogenecity" means a state in which the action of recognizing foreign substances and inducing immune rejection is significantly reduced. For example, when low immunogenic cells are introduced (e.g., transplanted) into the body of a recipient, immune rejection, which is normally induced by the recipient's recognition of the cells as non-autologous, does not occur or even if it does occur, is suppressed to a low level. Therefore, the "low immunogenic retinal pigment epithelial cell" of the present invention significantly suppresses induced immune rejection even when allogeneically transplanted into a recipient.

In the present invention, "pluripotent stem cell" refers to a stem cell capable of being cultured in vitro and having a potency to differentiate into any cell lineage belonging to three germ layers (ectoderm, mesoderm, endoderm) (pluripotency). Pluripotent stem cell can be induced from fertilized egg, clone embryo, germ stem cell, stem cell in a tissue, and the like. Examples of the pluripotent stem cell include embryonic stem cell (ES cell), EG cell (embryonic germ cell), induced pluripotent stem cell (iPS cell) and the like.

ES cell was first established in 1981, and has also been applied to the generation of knockout mouse since 1989. In 1998, human ES cell was established, which is also being utilized for regenerative medicine. ES cell can be produced by culturing an inner cell mass on a feeder cell or in a medium containing LIF. The production methods of ES cell are described in, for example, WO 96/22362, WO 02/101057, US 5,843,780, US 6,200,806, US 6,280,718 and the like. ES cells are available from given organizations, or a commercially available product can be purchased. For example, human ES cells, KhES-1, KhES-2 and KhES-3, are available from Kyoto University's Institute for Frontier Medical Sciences. EB5 cell, which is a mouse ES cell, is available from National research and development agency RIKEN, and D3 cell strain, which is a mouse embryonic stem cell, is available from ATCC.

Nuclear transfer ES cell (ntES cell), which is one of the ES cells, can be established from a clone embryo produced by transplanting the nucleus of a somatic cell into an egg from which a cell kabu is removed.

The "induced pluripotent stem cell" (also called iPS cell or iPSC cell) in the present invention is a cell induced to have pluripotency by reprogramming a somatic cell by a known method and the like. Specifically, a cell induced to have pluripotency by reprogramming differentiated somatic cells such as fibroblast, peripheral blood mononuclear cell and the like by the expression of a combination of a plurality of genes selected from the group consisting of reprogramming genes including Oct3/4, Sox2, Klf4, Myc (c-Myc, N-Myc, L-Myc), Glis1, Nanog, Sall4, lin28, Esrrb and the like can be mentioned. Examples of preferable combination of reprogramming factors include (1) Oct3/4, Sox2, Klf4, and Myc (c-Myc or L-Myc), and (2) Oct3/4, Sox2, Klf4, Lin28 and L-Myc (Stem Cells, 2013; 31:458-466) and the like.

iPS cell was established by Yamanaka et al. in mouse cell in 2006 (Cell, 2006, 126(4), pp.663-676). In 2007, iPS cell was also established from human fibroblast, and has pluripotency and self-renewal competence similar to those of ES cells (Cell, 2007, 131(5), pp.861-872; Science, 2007, 318(5858), pp.1917-1920; Nat. Biotechnol., 2008, 26(1), pp.101-106). Various improvements have thereafter been made in the induction method of iPS cells (e.g., mouse iPS cell: Cell. 2006 Aug 25; 126(4):663-76, human iPS cell: Cell. 2007 Nov 30; 131(5):861-72) .

Besides the production method of iPS cells based on direct reprogramming by gene expression, iPS cell can also be obtained from somatic cell by the addition of a compound and the like (Science, 2013, 341, pp. 651-654).

It is also possible to obtain established iPS cell and, for example, human induced pluripotent cell strains established by Kyoto University such as 201B7 cell, 201B7-Ff cell, 253G1 cell, 253G4 cell, 1201C1 cell, 1205D1 cell, 1210B2 cell, 1231A3 cell, Ff-I01 cell, QHJI01 cell and the like are available from the National University Corporation Kyoto University.

The pluripotent stem cell to be used in the present invention is preferably ES cell or iPS cell, more preferably iPS cell.

The pluripotent stem cells to be used in the present invention are preferably pluripotent stem cells of primates (e.g., human, monkey), more preferably human pluripotent stem cells. Therefore, the pluripotent stem cells to be used in the present invention are preferably human ES cells or human iPS cells, most preferably human iPS cells.

The "HLA" or "human leukocyte antigen" complex is a gene complex encoding MHC proteins in human. These cell surface proteins constituting the HLA complex are involved in the modulation of immune responses to antigens. In human, there are two MHCs of class I and class II, namely, "HLA-I" and "HLA-II". HLA-I includes three types of proteins (HLA-A, HLA-B, and HLA-C) that present peptides from the inside of the cell and attract killer T cells (also known as CD8⁺ T cells or cytotoxic T cells) by antigens presented by the HLA-I complex. The aforementioned HLA-I protein binds to B2M. HLA-II includes five proteins (HLA-DP, HLA-DM, HLA-DOB, HLA-DQ, and HLA-DR) that present antigens extracellularly to T lymphocytes. This stimulates CD4⁺ cells (also known as helper T cells). The use of either "MHC" or "HLA" has no limiting meaning since it depends on whether the gene is of human (HLA) or mouse (MHC) origin. Therefore, these terms may be used interchangeably in the present specification.

The "MHC class II-related gene" refers to a gene that encodes a protein involved in an immune response mediated by MHC class II. Therefore, for example, in the case of human, MHC class II is read as HLA class II, and HLA class II-related gene includes, in addition to genes encoding HLA class II molecules such as HLA-DM, HLA-DO, HLA-DP, HLA-DQ, and HLA-DR, and the like, genes encoding HLA class II regulatory proteins that regulate expression of HLA class II molecules, such as regulatory factor X-associated ankyrin-containing protein (RFXANK), regulatory factor v (RFX5), regulatory factor X-associated protein (RFXAP), and class II transactivator (CIITA), and the like. The sequences of any gene are available in publicly available databases.

A preferred MHC class II-related gene (HLA class II-related gene) in the present invention is a gene encoding CIITA.

The "MHC class I-related gene" refers to a gene encoding a protein involved in an immune response mediated by MHC class I. Therefore, for example, in the case of human, MHC class I is read as HLA class I, and HLA class I-related gene includes, in addition to genes encoding HLA class I molecules such as HLA-A, HLA-B, and HLA-C, and the like, genes encoding proteins that bind to and regulate the function of HLA class I molecules, such as β2 microglobulin (B2M) gene, TAP1 gene, TAP2 gene, and tapasin, and the like. The sequences of any gene are available in publicly available databases.

A preferred MHC class I-related gene (HLA class I-related gene) in the present invention is a gene encoding HLA-A, a gene encoding HLA-B, and a gene encoding HLA-C.

### 1. Method for producing low immunogenic retinal pigment epithelial cell (the production method of the present invention)

The present invention provides a method for producing a low immunogenic retinal pigment epithelial cell (low immunogeneic RPE cell), including the following steps:
step 1: a step of obtaining a pluripotent stem cell that does not express MHC class II protein or lacks the function thereof, and
step 2: a step of inducing an RPE cell from the pluripotent stem cell obtained in step 1.

### (step 1)

In step 1, pluripotent stem cells are obtained that do not express MHC class II protein, or express the protein but with impaired function of the protein (hereinafter also to be referred to as MHC class II-deficient pluripotent stem cell or low immunogenic pluripotent stem cell of the present invention). The functions of MHC class II protein that are impaired in the MHC class II-deficient pluripotent stem cells are those related to immunogenicity, such as antigen-presenting action.

The MHC class II-deficient pluripotent stem cell can be obtained, for example, by disrupting or modifying MHC class II-related genes.

The method of disrupting or modifying the MHC class II-related gene is not particularly limited as long as the MHC class II protein is not expressed or the function of the protein is impaired even if it is expressed. For example, homologous recombination technique can be used. A target gene on the chromosome can be modified by the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); Bio Manual series 8, gene targeting, Production of mutant mouse using ES cells, YODOSHA CO., LTD. (1995) and so on.

To be specific, for example, the genomic DNA comprising the target gene to be modified (MHC class II-related gene here) is isolated, and a targeting vector used for homologous recombination of the target gene is produced using the isolated genomic DNA. The produced targeting vector is introduced into stem cells and the cells that showed homologous recombination between the target gene and the targeting vector are selected, whereby stem cells having the modified gene on the chromosome can be produced.

Examples of the method for isolating genomic DNA comprising the target gene include known methods described in Molecular Cloning, A Laboratory Manual, Second Edition, Cold Spring Harbor Laboratory Press (1989), Current Protocols in Molecular Biology, John Wiley & Sons (1987-1997) and so on. The genomic DNA comprising the target gene can also be isolated using genomic DNA library screening system (manufactured by Genome Systems), Universal GenomeWalker Kits (manufactured by CLONTECH) and so on. A polynucleotide encoding the target protein can also be used instead of genomic DNA. The polynucleotide can beobtained by amplifying the corresponding polynucleotide by the PCR method.

A target vector used for homologous recombination of the target gene can be produced, and a homologous recombinant can be efficiently selected according to the methods described in Gene Targeting, A Practical Approach, IRL Press at Oxford University Press (1993); Bio Manual series 8, gene targeting, Production of mutant mouse using ES cells, YODOSHA CO., LTD. (1995) and so on. The target vector may be any of replacement type and insertion type, and the selection method may be positive selection, promoter selection, negative selection, polyA selection and so on.

As a method for selecting an intended homologous recombinant from the selected cell strains, Southern hybridization method, PCR method and so on for genomic DNA can be mentioned.

Genome editing technique can also be used as a method for disrupting or modifying MHC class II-related genes, and is a preferred embodiment. Genome editing technique refers to a technique that fuses a DNA cleavage domain to a system that recognizes the base sequence of target DNA. The present invention particularly intends a system that recognizes and binds to the base sequence of target DNA (MHC class II-related gene). Specifically, zinc-finger nuclease (ZFN), transcription activator-like effector nuclease (TALEN), clustered regularly interspaced short palindromic repeats (CRISPR)/CRISPR-associated proteins (Cas) CRISPR/Cas, hereinafter abbreviated as CRISPR/Cas), and the like can be mentioned.

ZFN is an artificial nuclease having zinc fingers as DNA-binding domains, and each zinc finger recognizes three bases. ZFN with 3 to 6 zinc fingers binds specifically to 9 to 18 base pairs (bp) and introduces DNA double-strand breaks with a specificity of 18 to 36 bp per pair.

TALEN is an artificial nuclease with a TALE which is possessed by the plant pathogenic bacterium Xanthomonas, as a DNA-binding domain. In the DNA-binding domain of TALE, a single unit of 34 amino acids that recognize a one base. TALENs with 15 to 20 units thereof are produced for each sense and antisense strand to introduce DNA double-strand breaks at the targeted sites.

The CRISPR/Cas system introduces a DNA double-strand break at the target base sequence by using a complex of a Cas nuclease that has DNA double-strand breaking activity and a target-sequence-specific single-stranded guide RNA. The CRISPR/Cas system has become a standard technique for genome editing around the world due to its convenience, high efficiency, and broad utility. Cas nuclease includes Cas9, Cas12a (Cpf1), Cas13a (C2c2), Cas14, Cas3, CasX, and the like, preferably Cas9. Cas9 includes Streptococcus pyogenes-derived Cas9 (SpCas9), Staphylococcus aureus-derived Cas9 (SaCas9), Francisella novicida-derived Cas9 (FnCas9), Campylobacter jejuni-derived Cas9 (CjCas9), and Streptococcus thermophilus-derived Cas9 (St1Cas9, St3Cas9). Cpf1 includes Acidaminococcus sp.-derived Cpf1 (AsCpf1) and Lachnospiraceae bacterium-derived Cpf1 (LbCpf1). Any genome editing tool may be used in the method of the present invention and, for example, the CRISPR/Cas9 system is preferred.

The CRISPR/Cas9 system, in one embodiment, is provided with a first vector containing a gene encoding a Cas9 protein and a second vector containing a guide RNA. In another embodiment, it can be a CRISPR/Cas9 vector system having a gene encoding Cas9 protein and a guide RNA in the same vector.

The guide RNA is appropriately designed to contain, in the 5' end region, a polynucleotide consisting of a base sequence complementary to the base sequence of preferably not less than 20 to not more than 24 bases, more preferably not less than 22 to not more than 24 bases, from 1 base upstream of the PAM sequence in the target double-stranded polynucleotide. Furthermore, it may contain one or more polynucleotides consisting of a base sequence that is non-complementary to the target double-stranded polynucleotide, arranged symmetrically about one point to form a complementary sequence, and that can form a hairpin structure.

A vector used in the CRISPR/Cas9 vector system is preferably an expression vector. As the expression vector, Escherichia coli-derived plasmids such as pBR322, pBR325, pUC12, pUC13, and the like; Bacillus subtilis-derived plasmids such as pUB110, pTP5, pC194, and the like; yeast-derived plasmids such as pSH19, pSH15, and the like; λphage and the like bacteriophage; viruses such as adenovirus, adeno-associated virus, lentivirus, vaccinia virus, baculovirus, cytomegalovirus, and the like; vectors modified from these, and the like can be used. Considering the activation of gene expression in vivo, viral vectors, particularly adeno-associated virus, are preferred.

In the aforementioned expression vector, promoters for expression of Cas9 protein and the aforementioned guide RNA are not particularly limited. For example, promoters for expression in animal cells such as EF1α promoter, SRα promoter, SV40 promoter, LTR promoter, CMV (cytomegalovirus) promoter, HSV-tk promoter and the like, promoters for expression in plant cells such as cauliflower mosaic virus (CaMV) 35S promoter and REF (rubber elongation factor) promoter, promoters for expression in insect cells such as polyhedrin promoter and p10 promoter, and the like can be used.

The aforementioned expression vector may further have multi cloning site, enhancer, splicing signal, polyA addition signal, selection marker (drug resistance) and promoter thereof, replication origin and the like.

An MHC class II-related gene can be site-specifically modified according to a method employed generally.

First, the aforementioned protein and the aforementioned guide RNA are mixed under mild conditions and incubated. The mild conditions refer to temperature and pH at which the protein is not degraded or denatured. The temperature is preferably not less than 4°C and not more than 40°C, and the pH is preferably not less than 4 and not more than 10. The incubation time is preferably not less than 0.5 hr and not more than 1 hr. The complex of the aforementioned protein and the aforementioned guide RNA is stable and can remain stable even after standing at room temperature for several hours.

Next, the aforementioned protein and the aforementioned guide RNA form a complex on the aforementioned target double-stranded polynucleotide. The aforementioned protein recognizes the PAM sequence and binds to the aforementioned target double-stranded polynucleotide at a binding site located upstream of the PAM sequence. Subsequently, a target double-stranded polynucleotide modified according to the purpose can be obtained in a region determined by complementary binding between the aforementioned guide RNA and the aforementioned double-stranded polynucleotide.

In the present specification, the "modification" means that the target double-stranded polynucleotide changes structurally or functionally. For example, structural or functional alteration of the target double-stranded polynucleotide due to the addition of a functional protein or base sequence can be mentioned. The modification enables modification, deletion, enhancement, or suppression of the function of the target double-stranded polynucleotide, and addition of a new function. The "modification" in the present invention intends a change that impairs the function of the target double-stranded polynucleotide.

The CRISPR/Cas9 vector system can be introduced into cells by a method suitable for the viable cells to be used. For example, electroporation method, heat shock method, calcium phosphate method, lipofection method, DEAE dextran method, microinjection method, particle gun method, method using virus, methods using commercially available transfection reagents such as FuGENE (registered trade mark) 6 Transfection Reagent (manufactured by Roche), Lipofectamine 2000 Reagent (manufactured by Invitrogen), Lipofectamine LTX Reagent (manufactured by Invitrogen), Lipofectamine 3000 Reagent (manufactured by Invitrogen), and the like, and the like can be mentioned.

Desired cells can be obtained by repeating passage of the cells thus obtained.

When the MHC class II-deficient pluripotent stem cell is commercially available or can be donated, step 1 corresponds to a step of obtaining the cell.

### (step 2)

In step 2, RPE cells are induced to differentiate from MHC class II-deficient pluripotent stem cells obtained from step 1. The step of inducing differentiation can be performed in the same manner as the method of inducing differentiation of pluripotent stem cells into RPE cells, which is generally practiced in the pertinent field. Specifically, differentiation can be induced by establishing pluripotent stem cells and culturing the cells in an RPE-specific medium supplemented with RPE differentiation factors (see reference documents below). Since RPE cells show the morphology of polygonal epithelial cells with brown pigment, it is possible to pick up clusters of cells with black pigment under a microscope and purify them.

### <reference documents>

Neurosci Lett. 2009 Jul 24; 458(3):126-31. doi: 10.1016/j.neulet.2009.04.035. Epub 2009 Apr 18.
Proc Natl Acad Sci U S A. 2002 Feb 5; 99(3):1580-5. doi: 10.1073/pnas.032662199. Epub 2002 Jan 29.
PLoS One, 8: 409-412, 2011 Invest Ophthalmol Vis Sci 2015 Jan 20; 56(2):1051-62
Stem Cell Reports Volume 7, Issue 4, 11 October 2016, 619-634 WO2005/070011, WO2006/080952, WO2011/063005, W2009/051671, US2011269173, WO2011028524, WO2015/053375, WO2015/068505, WO2017/043605

### 2. Low immunogenic retinal pigment epithelial cell (low immunogenic RPE cell of the present invention)

The low immunogenic RPE cell of the present invention does not express or lacks the function of MHC class II proteins (human leukocyte antigen (HLA) class II protein in the case of human). As one example, the cells produced by the production method of the present invention described in the above-mentioned 1 can be mentioned, and RPE cells obtained by inducing differentiation from MHC class II-deficient pluripotent stem cells can be mentioned. The low immunogenic RPE cell of the present invention is characterized by low immunogenicity compared to RPE cells differentiated from pluripotent stem cells in which MHC class II-related genes are not disrupted or modified (i.e. unmanipulated pluripotent stem cells).

On the other hand, the low immunogenic RPE cell of the present invention preferably expresses MHC class I proteins (human leukocyte antigen (HLA) class I protein in the case of human), and do not lack the functions thereof. One example is an embodiment in which MHC class I-related gene (HLA class I-related gene) is retained intact. MHC class I-related genes that are preferably retained include HLA-A, HLA-B, and HLA-C.

### 3. Transplant material containing the low immunogenic RPE cell of the present invention or RPE cell induced from low immunogenic pluripotent stem cell (transplant material of the present invention)

The low immunogenic RPE cell of the present invention is the same as that described in the above-mentioned 2. RPE cell induced from the low immunogenic pluripotent stem cell of the present invention is the same as that described in the above-mentioned 1. These RPE cells can be used as transplant materials, particularly, low immunogenic transplant materials. The RPE cell of the present invention can be used as a biological material for transplantation, which is used for supplementing a damaged cell or disordered tissue itself in a cell damage state (e.g., used for transplantation operation) and so on.

When a transplantation material containing the RPE cell of the present invention is produced as a cell sheet, the pluripotent stem cell of the present invention are seeded on a culture substrate and cultured in a differentiation-inducing medium to induce differentiation into RPE cells on the culture substrate. The culture substrate used in the present invention is not particularly limited as long as it is for cell culture. For example, polymer materials derived from naturally occurring substances such as collagen, gelatin, cellulose, laminin, and the like, synthetic polymer materials such as polystyrene, polyester, polycarbonate, poly(N-isopropylacrylamide), and the like, biodegradable polymer material such as polylactic acid, polyglycolic acid and the like, hydroxyapatite, amniotic membrane, and the like can be mentioned. Preferably, one that does not cause rejection at the time of transplantation is appropriately determined according to the transplant recipient.

Examples of the transplantation method include, but are not limited to, the methods described in Examples below.

The transplantation material of the present invention is preferably used for treating age-related macular degeneration and retinal pigment denaturation for which transplantation of RPE cells is an effective treatment method, RPE failure which is a group of diseases that causes RPE abnormality and retinal degeneration associated therewith such as crystalline retinopathy, Leber's congenital black cataract, high myopia, polypoidal retinal pigment epitheliopathy, pigment striations, Best disease, Stargardt disease, choroideremia and the like,

Other features of the present invention will become clear in the course of the following description of exemplary embodiments given for the purpose of explanation of the present invention; however, they do not intend to limit the present invention.

### [Example]

### Example 1 Allogeneic transplantation of monkey MHC class II-deficient ES cell-derived RPE cell and immunoresponse test

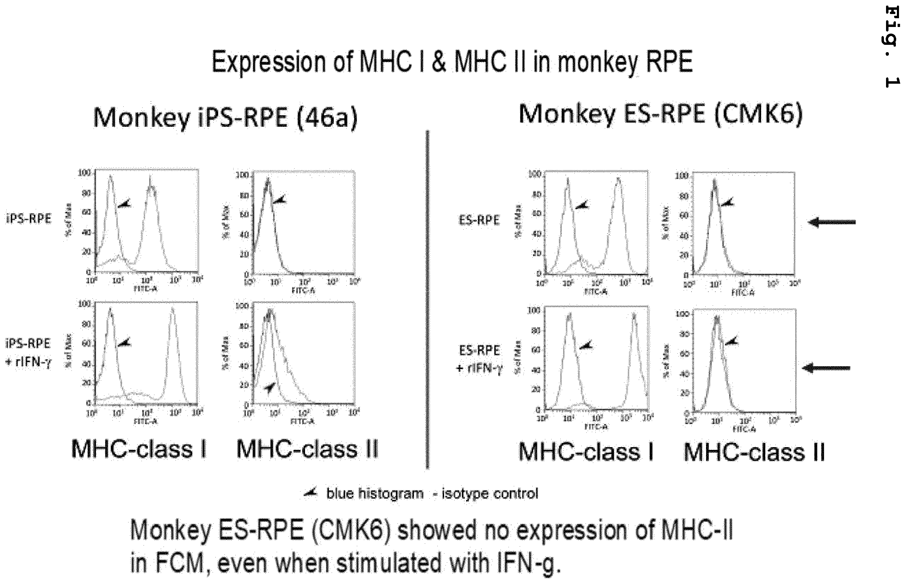

RPE cell was produced by inducing differentiation of spontaneous MHC class II-deficient monkey ES cell CMK6 strain (CMK6 ES-RPE cell). MHC class II deficiency was confirmed by flow cytometry analysis (Fig. 1).

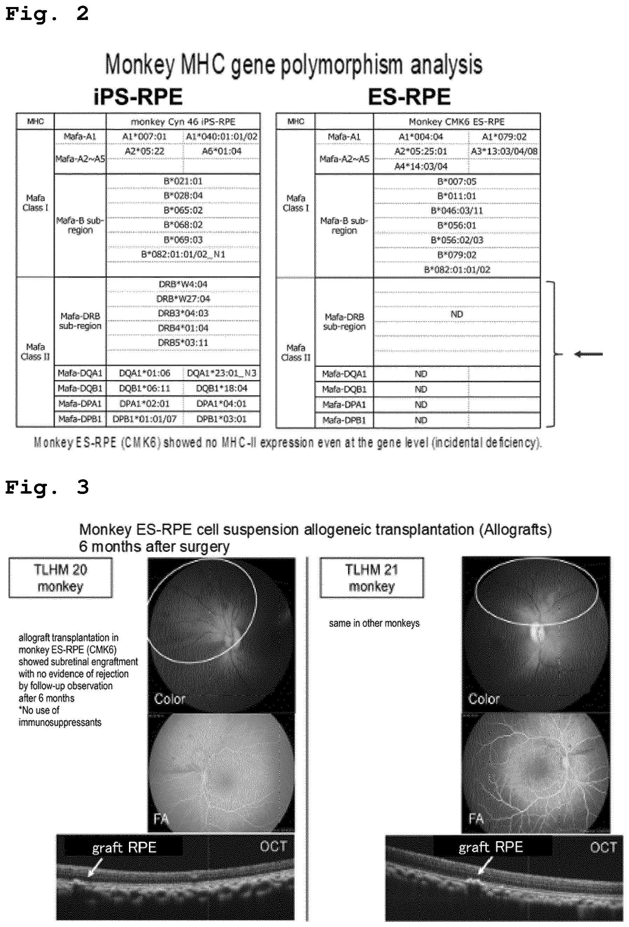

MHC class II deficiency was also confirmed by monkey MHC gene polymorphism analysis (Fig. 2).

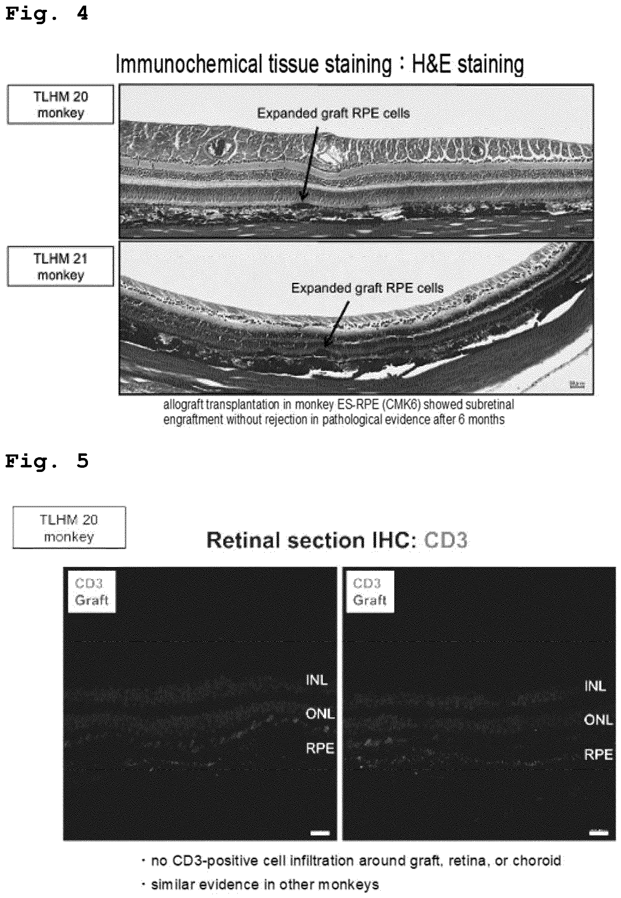

The above-mentioned CMK6 ES-RPE cells were allogeneically transplanted into normal Macaca fascicularis (Cyn51). Engraftment was confirmed 6 months after the operation (Fig. 3), and graft engraftment was also confirmed by H&E staining of a retinal section (Fig. 4). Immunostaining of retinal sections showed no CD3-positive T cell infiltration into the retina (Fig. 5) .

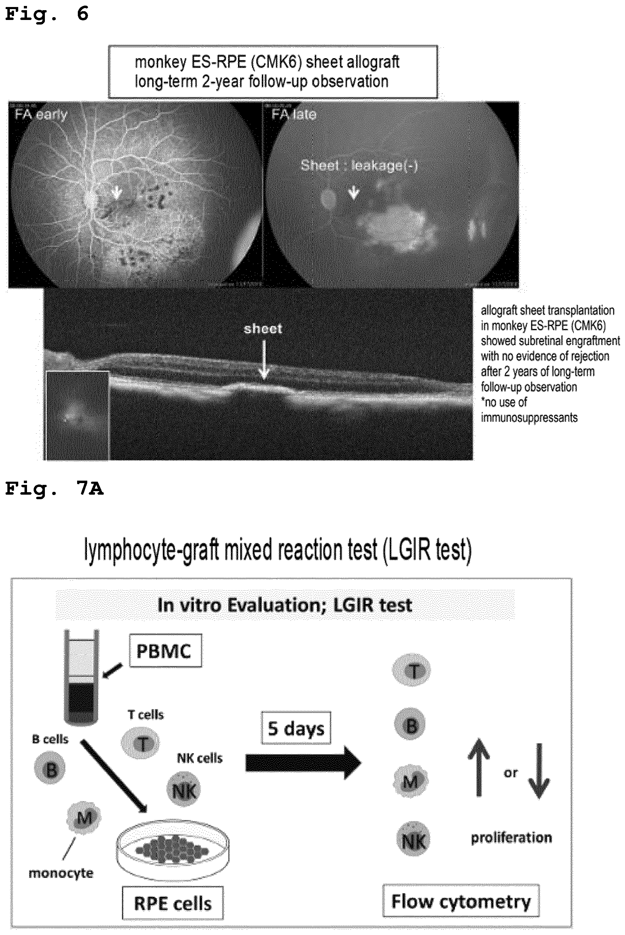

CMK6 ES-RPE cells were allogeneically transplanted into normal Macaca fascicularis. Long-term engraftment was confirmed 2 years after the operation (Fig. 6).

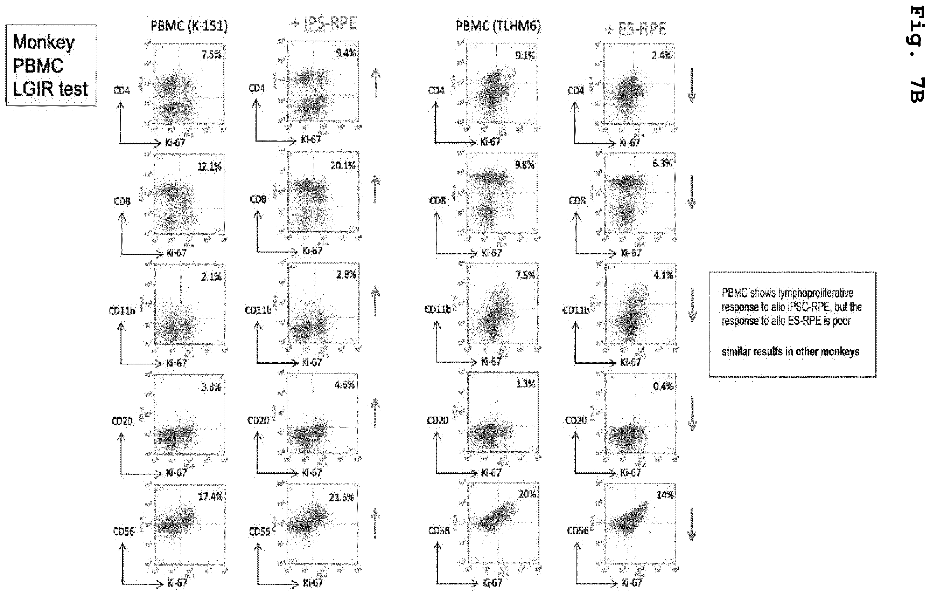

CMK6 ES-RPE cells were unresponsive to negative - T, B, NK cells and monocytes in a lymphocyte graft immune response test (LGIR test) (Fig. 7).

On the other hand, monkey iPS cell-derived RPE cells (46a strain) expressed MHC class II, and an alloimmune response was observed.

### [Experimental Example 1 (Fig. 1)]

### Expression of MHC class I and class II in iPSC/ESC-derived RPE cells

Expression of MHC molecule in monkey iPS-RPE cells and ES-RPE cells was examined by flow cytometry analysis. iPS-RPE cell strain 46a constitutively expressed MHC class I, and iPS-RPE cells pretreated with interferon-γ (IFN-γ) expressed MHC class II (left Figure). On the other hand, ES-RPE cell strain CMK6 constitutively expressed MHC class I, and IFN-γ-treated ES-RPE cells failed to express MHC class II on the surface thereof (right Figure).

### [Experimental Example 2 (Fig. 2)]

### MHC typing in monkey iPSC/ESC-derived RPE cells

The results of MHC allele typing in monkey iPS-RPE cells and ES-RPE cells are shown in Table (Fig. 2). Genotyping of MHC class I (Mafa class I) and class II (Mafa class II) genes of M. fascicularis (cynomolgus monkey) was performed by the pyrosequencing method as described previously (Shiina et al, Immunogenetics volume 67, pages 563-578 (2015) 2015). As a result, the iPS-RPE cell strain 46a exhibited a heterozygous MHC phenotype (left Figure). On the other hand, the ES-RPE cell strain CMK6 exhibited a heterozygous MHC class I phenotype, but did not express MHC class II at the mRNA level (right Figure). This suggests that this monkey ES-RPE cell strain was spontaneously deficient in MHC class II.

### [Experimental Example 3 (Fig. 3)]

### Monkey-derived ES-RPE cells are poorly immunogenic in in vivo animal models

CMK6 ES-RPE cells were transplanted into the eyes of MHC-mismatched donor monkeys (2 cynomolgus monkeys without immunosuppression). The transplanted grafts were observed by color fundus photography, fluorescein angiography (FA), and optical coherence tomography (OCT) 6 months after surgery. As a result, no sign of rejection was observed in the allograft of the MHC-mismatched monkey (CMK6 ES-RPE cells transplanted into TLHM20 monkey eye: left Figure). Similarly, MHC-mismatched monkey allografts (CMK6 RPE cells transplanted into TLHM21 monkey eyes) showed no rejection, and the grafts appeared to have been engrafted in the subretinal space of the eye (right Figure). The present inventors previously showed that when iPS-RPE cells of MHC-mismatched monkey were used, MHC-mismatched donor/recipients experienced a severe immune attack on the retina and no graft survived at the time point of 6 months (reference documents).

### <reference documents>

Stem Cell Reports. 2016 Oct 11; 7(4):635-648. doi: 10.1016/j.sterner.2016.08.010. Epub 2016 Sep 15.
Stem Cell Reports. 2017 Nov 14; 9(5):1501-1515. doi: 10.1016/j.sterner.2017.10.003. Epub 2017 Nov 5.

### [Experimental Example 4 (Fig. 4)]

In addition, the presence of inflammatory cells in the monkey ES-RPE cell-implanted model was histologically examined by H&E staining and inflammatory cell immunohistochemistry (IHC) of paraffin sections. For IHC, TLHM20, 21 monkey retinas were stained with anti-CD3 antibody. H&E staining showed that the RPE cells engrafted in these monkeys were located in the subretinal space, where the engrafted RPE cells proliferated, indicating that the allografts had no sign of immune rejection.

### [Experimental Example 5 (Fig. 5)]

As a result of IHC analysis, it was found that CD3⁺ cells (T-cell marker) were not present in the retina, subretinal space, or choroid in TLHM20 monkey retinal sections. Similar data were obtained in retinal sections of TLHM21 monkeys (data not shown).

### [Experimental Example 6 (Fig. 6)]

### Long-term survival of monkey ES-RPE cell sheet in in vivo animal model

Then, CMK6 ES-RPE cell sheet was transplanted into the eyes of MHC-mismatched donor monkeys (cynomolgus monkeys without immunosuppression). Transplanted grafts were observed by color fundus, FA, and OCT 2 years after surgery. As a result, no sign of rejection was observed in the MHC-mismatched monkey allografts (FA finding: no leakage from sheet), and the transplanted sheet appeared to have engrafted in the subretinal space at the time of the second year observation (OCT finding).

### [Experimental Example 7 (Fig. 7)]

### LGIR assay method using RPE cells

In order to examine the immunogenicity of ES- and iPS-RPE cells, monkey peripheral blood mononuclear cells (PBMC) were prepared from the blood of healthy donors (Macaca fascicularis). PBMC (2.5×10⁶) were co-cultured with ES-RPE cells (CMK6) or iPS-RPE cells (46a) in RPMI1640 medium containing recombinant interleukin-2 (IL-2) in 24-well plates for 4-5 days. In the LGIR assay, PBMCs were harvested from co-culture dishes and stained with PE-labeled anti-Ki-67 antibody and APC-labeled primary antibody (CD4⁺ T cells, CD8⁺ T cells, CD20⁺ B cells, CD56⁺ or NKG2A⁺ NK cells, CD11b⁺ monocytes) at 4°C for 30 min. Evaluation of immunogenicity of ES- and iPS-RPE cells was performed with Ki-67 FACS (reference documents).

### <reference documents>

Stem Cell Reports. 2016 Oct 11; 7(4):619-634. doi: 10.1016/j.stemcr.2016.08.011. Epub 2016 Sep 15.
J Clin Med. 2020 Jul 13; 9(7):2217. doi: 10.3390/jcm9072217.
Prog Retin Eye Res. 2021 Jan 19:100950. doi: 10.1016/j.preteyeres.2021.100950.

### Immunogenicity of monkey-derived ES-RPE cells and iPS-RPE cells to allogeneic PBMC (LGIR assay)

Whether this activation was due to an immune response was confirmed by directly comparing the proliferation of ES/iPS-RPE cells with MHC-mismatched allogeneic PBMC. In the MHC mismatch LGIR assay, co-culture with 46a iPS-RPE cells increased proliferation of immune cells such as CD4⁺ T cells, CD8⁺ T cells, CD11b⁺ monocyte, CD20⁺ B cells, CD56⁺ NK cell and the like (Fig. 7B, left Figure). On the other hand, co-culture with allogeneic ES-RPE cells (CMK6: Fig. 7B, right Figure) reduced the proliferation of these immune cells. It was suggested that monkey iPS-RPE cells have sufficient immunogenicity to elicit sufficient allogeneic immune response, whereas monkey ES-RPE cells that do not express MHC-II lacks immunogenicity.

### Example 2 Allogeneic transplantation of monkey MHC class II knockout (KO) iPS cell-derived RPE cell and immune response test

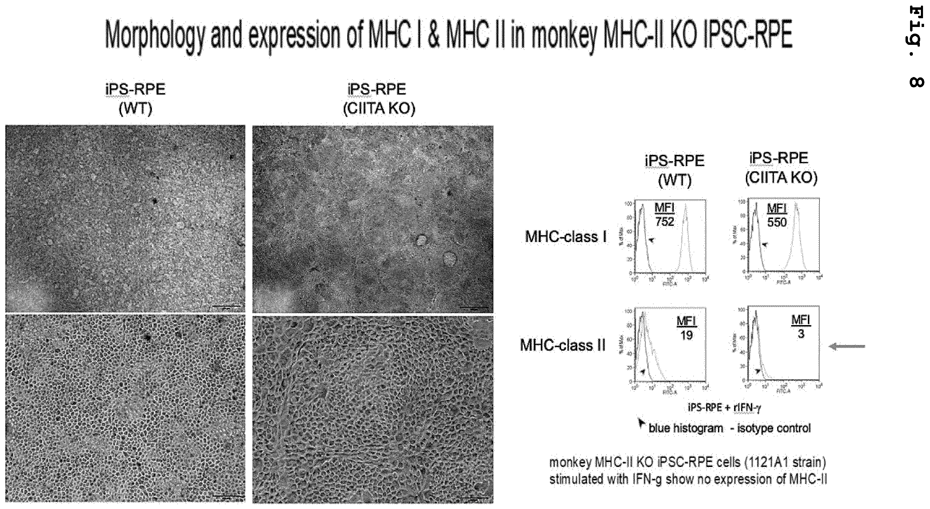

Cas9 and gRNAs for CIITA were introduced into two types of iPS cell strains 1121A1 and 46a by lipofection to prepare MHC class II-deficient strains, which were induced to differentiate into RPE cells. MHC class II deficiency was confirmed by flow cytometry analysis (Fig. 8).

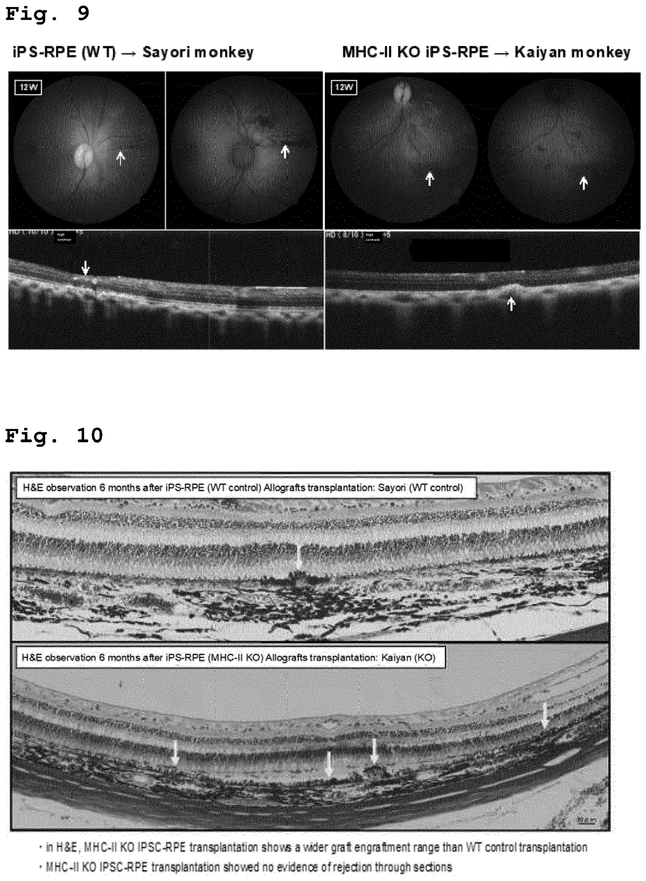

The monkey MHC class II KO iPS-RPE cells (1121A1 strain) produced were allogeneically transplanted into both eyes of normal Macaca fascicularis (HM-23). Engraftment was confirmed 3 months after surgery (12W). On the other hand, when control WT iPS-RPE cells (1121A1 strain) were allogeneically transplanted into both eyes of normal Macaca fascicularis (HM-22), mixed findings of engraftment and rejection were observed (Fig. 9).

Engraftment was confirmed by H&E staining 6 months after surgery (Fig. 10).

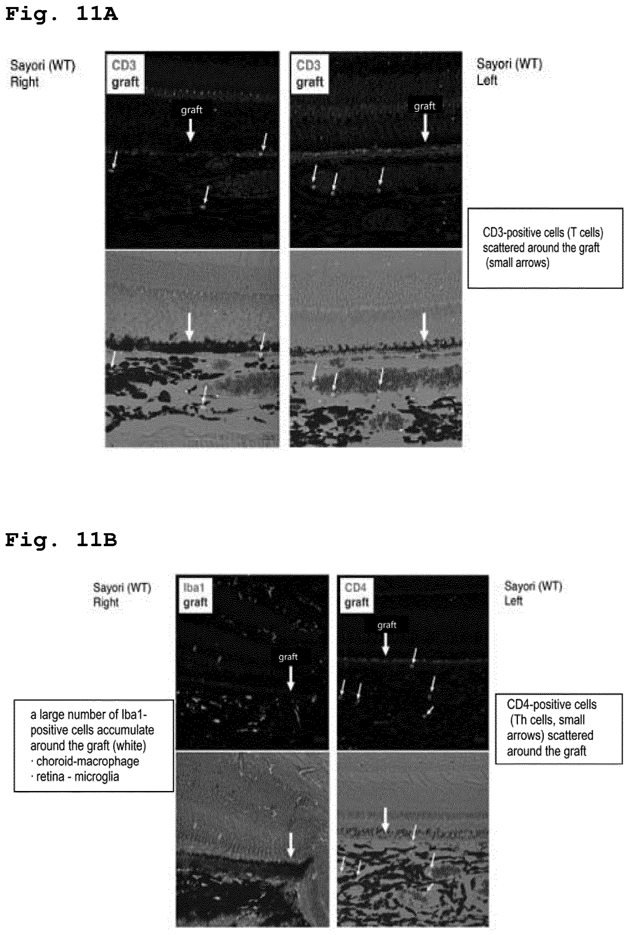

Immunostaining of retinal sections after transplantation of wild-type (WT) monkey iPS cell-derived RPE cells (1121A1 strain) showed CD3-positive T-cell infiltration, CD4-positive T-cell infiltration, and Iba1-positive microglia-macrophage infiltration in the retina = rejection findings were obtained (Fig. 11A, Fig. 11B).

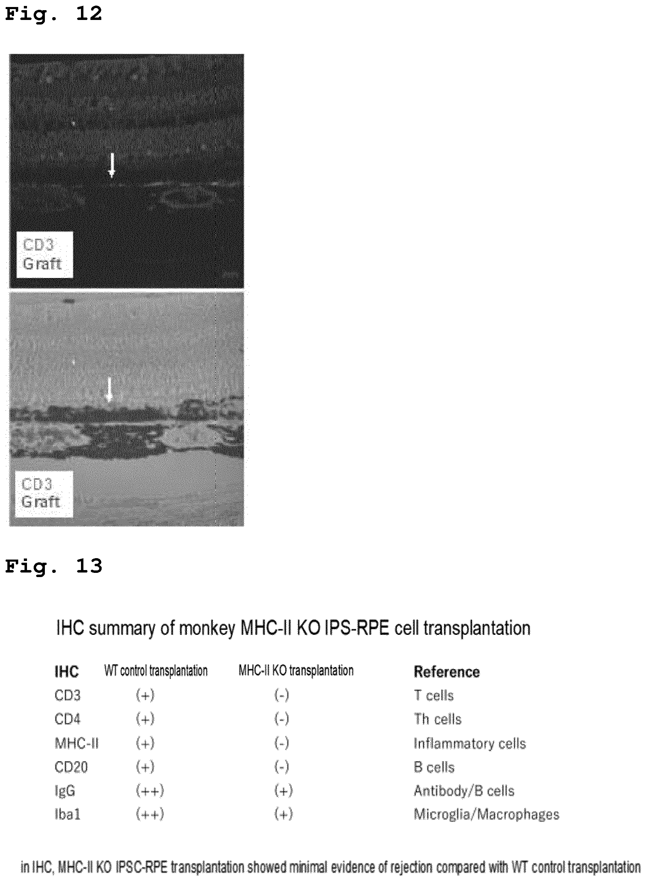

Immunostaining of retinal sections after transplantation of KO (Class II KO) monkey iPS cell-derived RPE cells (1121A1 strain) showed no infiltration of CD3-positive T cells in the retina = no rejection (Fig. 12).

Summary of immunostaining of retinal sections after transplantation of WT and MHC class II KO monkey iPS cell-derived RPE cells (1121A1 strain). In the KO-iPS RPE cell transplantation, intraocular inflammation findings were clearly milder than in WT (Fig. 13).

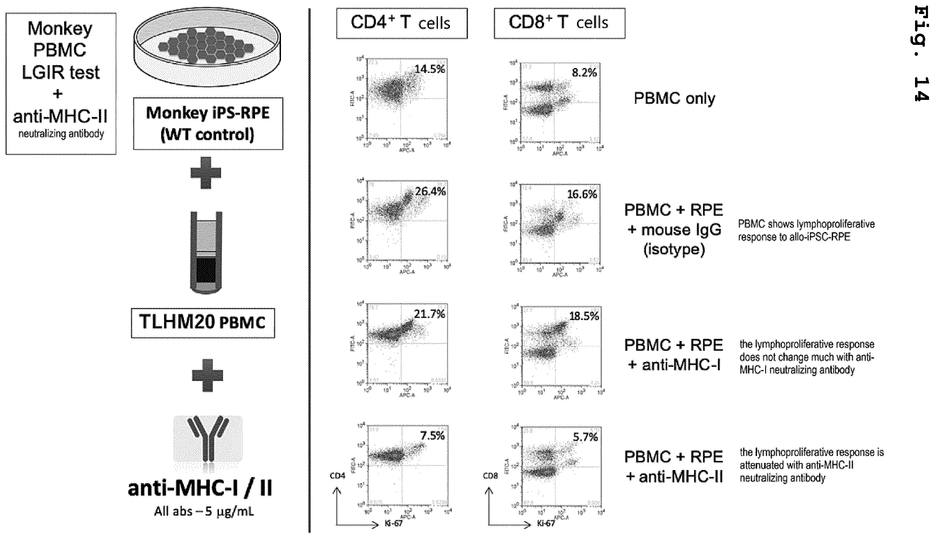

In the lymphocyte graft immune response test (LGIR test), an alloimmune response (T cell response) was observed against WT iPS-RPE cells, but the reaction was attenuated when an MHC-II neutralizing antibody was added. On the other hand, the MHC-I neutralizing antibody failed to block (Fig. 14).

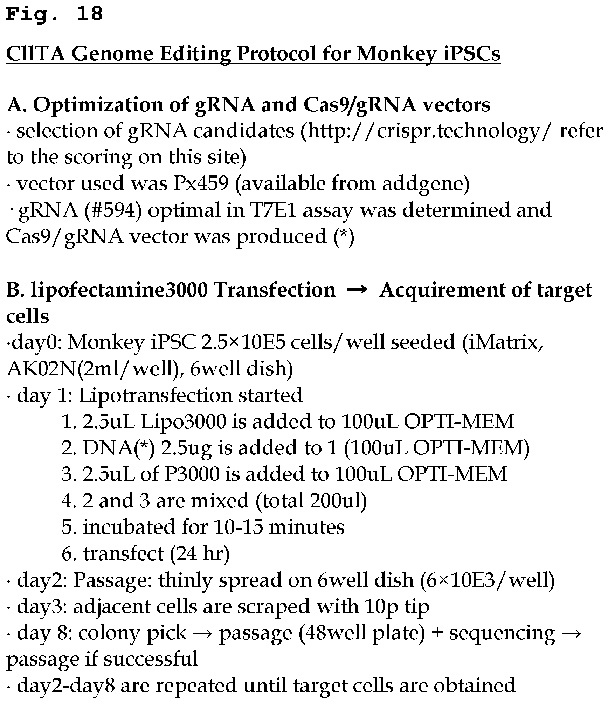

### [Experimental Example 8 (Fig. 8 and Fig. 18)]

Establishment of monkey MHC-II knockout iPSC-derived RPE cells As the next step, RPE cells were established as a transplant material from monkey MHC homozygous iPSCs. Monkey 1PSC1121A1, which is an MHC homozygote, was established from skin fibroblasts using an episomal vector as previously reported (reference document). Next, MHC-II knockout iPSC-derived RPE cells (1121A1 strain) were established: Cas9 and gRNAs against CIITA were introduced into iPS cell line 1121A1 by lipofection (see the following sequences) to generate MHC class II deficient cells, and then differentiated into RPE cells. Genome editing protocol is shown in Fig. 18. MHC class II KO RPE cell is a pigmented cell having a hexagonal morphology similar to that of wild-type (WT) iPS-RPE cells (Fig. 8, left Figure).
CIITA_hs594 (grID: hs044825594): ATAGGACCAGATGAAGTGATCGG (SEQ ID NO: 1)

MHC class II deficiency in RPE cells was confirmed by flow cytometry analysis (Fig. 8, right Figure). As expected, MHC class II KO RPE cells did not express MHC-II molecules even when IFN-γ was added, whereas WT control RPE cells expressed MHC-II on the surface.

### <reference documents>

Stem Cell Reports 2016 Oct 11; 7(4):635-648.
Stem Cell Reports 2017 Nov 14; 9(5):1501-1515

### [Experimental Example 9 (Fig. 9)]

### Immunohistochemistry of MHC-II KO iPS-RPE cell transplantation in in vivo animal model

MHC-II KO iPS-RPE cells or control WT RPE cells were transplanted into the eyes of MHC-mismatched donor (Macaca fascicularis without immunosuppression). The transplanted grafts were observed by color fundus photography, fluorescein angiography (FA), and optical coherence tomography (OCT) 3 months after surgery. As a result, sign of rejection was observed in the allograft of the control (WT RPE cell) MHC-mismatched monkey (Sayori monkey: left Figure). On the other hand, MHC-II KO iPS-RPE allograft of MHC-mismatched monkey (Kaiyan monkey) showed no sign of rejection, and the graft appeared to have been engrafted in the subretinal space of the eye (right Figure).

### [Experimental Example 10 (Fig. 10)]

Transplanted grafts were evaluated by H&E staining and IHC 6 months after surgery. As a result, no sign of rejection was observed in MHC-II KO iPS-RPE allografts of MHC-mismatched monkey (Kayan monkey). When compared with data from WT RPE cell transplantation (Sayori monkey), H&E-stained paraffin sections showed expansion of graft into the subretinal space.

### [Experimental Example 11 (Fig. 11)]

In IHC, the retina of Sayori/Kaiyan monkey was stained with anti-CD3 antibody. As a result of IHC analysis, multiple CD3⁺ cell infiltration (T cell markers) was confirmed in the retina, subretinal space, and choroid through sections of the retina of Sayori monkeys (Fig. 11A). Similar data were obtained for Iba1 (microglia/macrophage) and CD4 (helper T cell) (Fig. 11B). In addition, retinal sections were stained with MHC-II (inflammatory cells), CD20 (B cells), and IgG (antibody/B cells), indicating infiltration of inflammatory immune cells into the retina (data not shown).

### [Experimental Example 12 (Fig. 12)]

On the other hand, infiltration of CD3⁺ T cells into the retina, subretinal space, or choroid was not observed in the retinal sections of Kaiyan monkey (MHC-II KO transplantation).

### [Experimental Example 13 (Fig. 13)]

Fig. 13 summarizes the IHC of MHC-II KO transplantation in comparison with the data of control WT transplantation. As is clear from Fig. 13, MHC-II KO transplantation was found to cause less immune attack than control transplantation.

### [Experimental Example 14 (Fig. 14)]

### LGIR assay with anti-MHC class II blocking antibody

In MHC mismatch LGIR assay using WT control iPS-RPE cells expressing MHC-II, proliferation of T cells (CD4⁺ & CD8⁺ T cells) increased as compared with PBMC alone without RPE cells. On the other hand, when co-cultured with RPE cells in the presence of anti-MHC-II blocking antibody, the proliferation of these T cells did not respond. Interestingly, T cell proliferation was also responsive in the presence of anti-MHC-I blocking antibody, suggesting that MHC-II molecules on RPE cells, not MHC-I molecule, have immunogenicity for eliciting a sufficient allogeneic immune response.

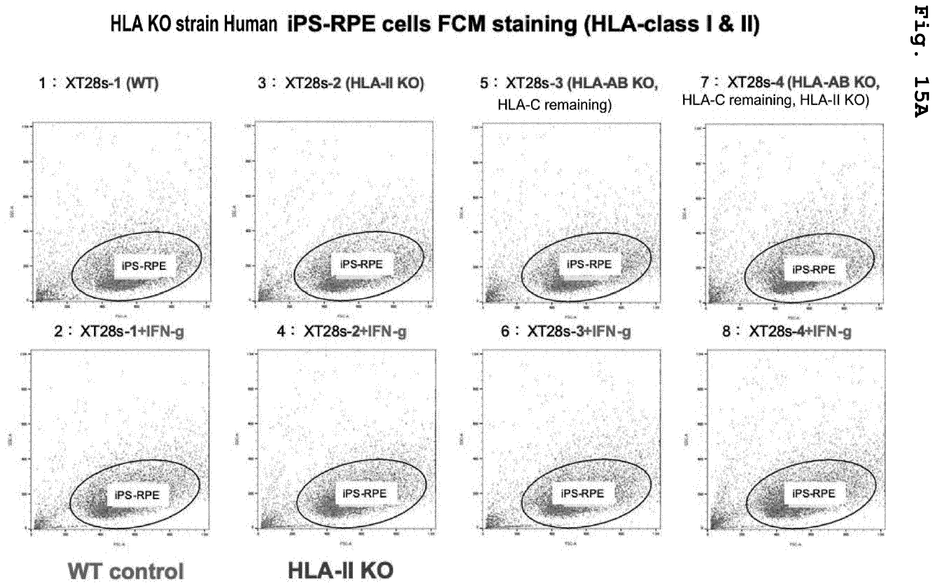

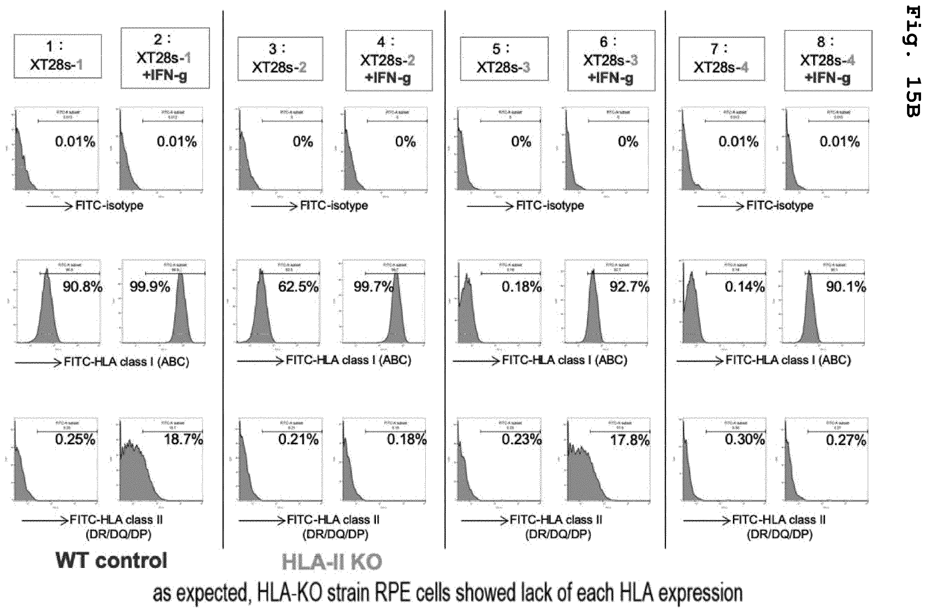

### [Experimental Example 15 (Fig. 15)]

### Expression of HLA class I and class II in human HLA KO iPS-RPE cells

The expression of HLA molecules in human HLA KO iPS-RPE cells was examined by flow cytometry analysis. Four iPS-RPE cell strains were used for the assay. (1) WT control (WT28s-1), (2) HLA class II KO (WT28s-2, (3) HLA class I KO (WT28s-3), (4) HLA class I & II KO (WT28s-4).

WT iPS-RPE cells constitutively expressed HLA class I, and WT iPS-RPE cells pretreated with interferon-γ (IFN-γ) expressed HLA class II (Fig. 15A). On the other hand, HLA class II KO cells constitutively express HLA class I, and RPE cells treated with IFN-γ fail to express HLA class II on the surface. In addition, HLA class I KO RPE cells could not express HLA class I on the surface. Furthermore, class I and class II double KO RPE cells did not express both HLA molecules. Thus, the expression of HLA molecules in human HLA KO RPE cells could be confirmed as described (Fig. 15B).

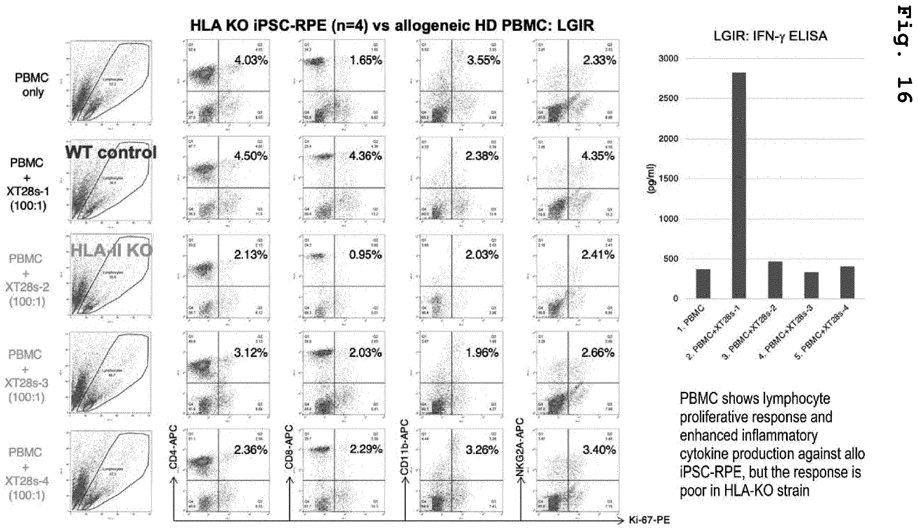

### [Experimental Example 16 (Fig. 16)]

### Low immunogenicity of human HLA KO iPS-RPE cells (LGIR assay)

The immunogenicity of human HLA KO iPS-RPE cells was examined by LGIR in vitro assay. In LGIR assay using WT control RPE cells, the proliferation of immune cells, including CD4⁺ T cells, CD8⁺ T cells, CD11b⁺ monocytes, and CD56⁺ NK cells, increased as compared with PBMC alone without containing RPE cells. On the other hand, co-culture with HLA KO RPE cells (class II KO, class I KO, double KO) decreased the proliferation of these immune cells. Similarly, the supernatant of PBMC-HLA KO RPE cell co-culture contained low concentrations of the inflammatory cytokine IFN-γ by ELISA, whereas the supernatant of PBMC-WT RPE cell co-culture contained high concentrations of IFN-γ (right Figure).

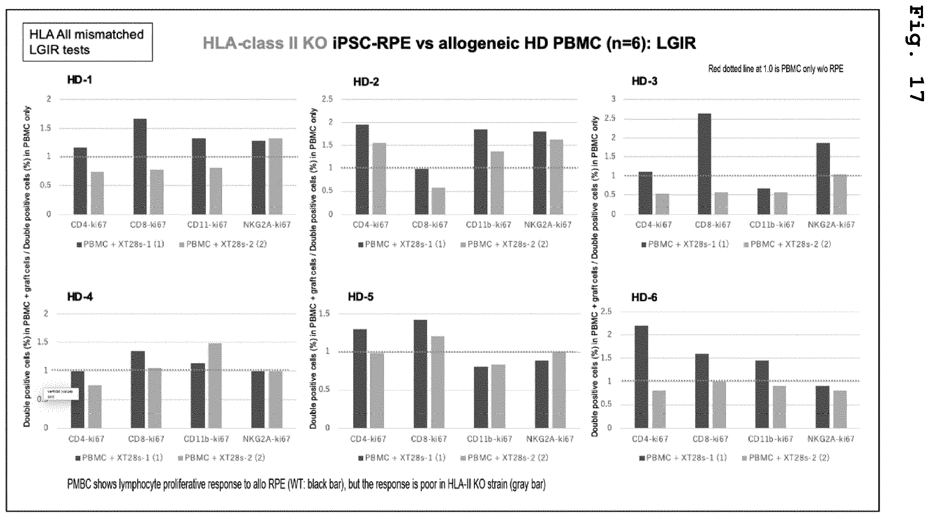

### [Experimental Example 17 (Fig. 17)]

### Low immunoresponsiveness of human HLA class II KO iPS-RPE cells (LGIR assay)

Next, the immunogenicity of human HLA class II KO iPS-RPE cells was confirmed by LGIR in vitro assay using multiple PBMC donors (n=6). As compared with the data of WT control (black bar), co-culture with HLA class II KO RPE cells (grey bar) increased the proliferation of immune cells such as CD4⁺ T cells, CD8⁺ T cells, CD11b⁺ monocytes, CD56⁺ NK cells, and the like. These data suggest that iPS-RPE cells have immunogenicity sufficient to elicit allogeneic immune responses, whereas HLA KO RPE cells, which do not express HLA molecules, particularly HLA class II, do not.

### Example 3 Production of human HLA class II-deficient iPS cell-derived RPE cell, immune response test

The following three types of HLA-deficient iPS cell-derived RPE cells were produced.
1: WT28s-1 RPE (Ff-XT28s05 strain (Cell stem Cell. 2019; 24 566-578)-derived RPE cell, hereinafter WT control strain)
2: WT28s-2 RPE, HLA class I mono-allele KO, class II KO (Ff-XT28s05-MTo #2 strain (same as above)-derived RPE cell, hereinafter HLA-II KO strain)
3: WT28s-3 RPE, HLA-A, B KO, HLA-C remaining (Ff-XT28s05-ABo #14 strain (same as above)-derived RPE cell, hereinafter HLA-I KO strain)
4: WT28s-4 RPE, HLA-A, B KO, HLA-C remaining, class II KO (Ff-XT28s05-ABo_To # 14-4 strain (same as above)-derived RPE cell, hereinafter HLA-I & II KO strain)

Each HLA deficiency was confirmed by flow cytometry analysis (Fig. 15).

The WT control strain is positive, and thus responded to T cells, NK cells, and monocytes in the lymphocyte graft immune response test (LGIR test). On the other hand, no immune response was observed with any of the three types of HLA-deficient iPS cell-derived RPE cells (Fig. 16).

In the LGIR test, the reaction with the WT control strain and the reaction with the HLA-II KO strain (PBMCs derived from 6 healthy subjects were prepared) was compared. Alloimmune responses against WT iPS-RPE cells were observed (various immune cell responses), but the responses were clearly attenuated in the HLA-II KO strain (Fig. 17).

The above results suggest that HLA class II-deficient RPE cells have the effect of suppressing post-transplantation immune responses.

### Example 4 Allogeneic transplantation of monkey MHC class II knockout (KO) iPS cell-derived RPE cell

The monkey MHC class II KO iPS-RPE cells (1121A1 strain) prepared in Example 2 were allogeneically transplanted into the right eye of normal Macaca fascicularis (HM-24). No immunosuppressants were used.

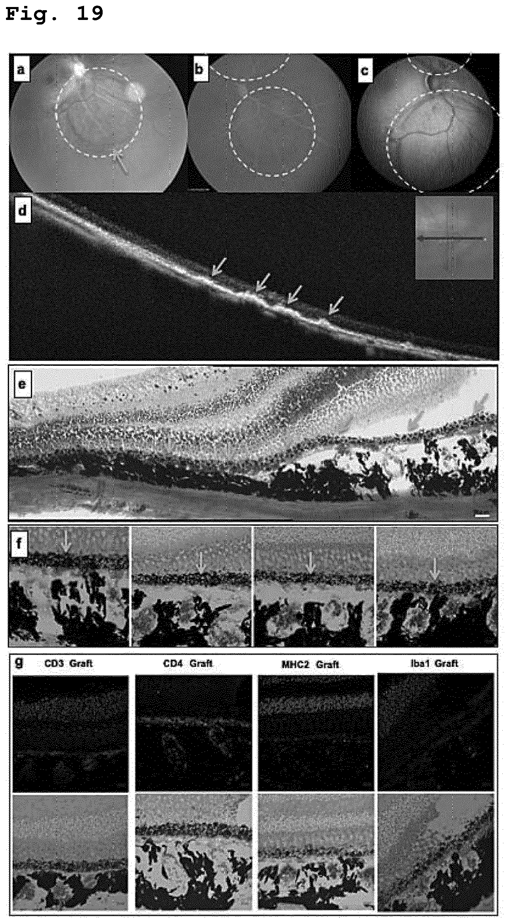

At 6 months after surgery, grafts were confirmed by color fundus photography (a), fluorescein angiography (FA) (b), fundus autofluorescence (FAF) (c), and optical coherence tomography (OCT) (d) (Fig. 19). As a result, no rejection was found by MHC class II KO iPS-RPE allografts (a, b, c). Engraftment of RPE allograft was also observed in the subretinal space (d). Grafts were also confirmed by H&E staining 6 months after surgery (e). No rejection was seen by MHC class II KO iPS-RPE allografts in monkey. Furthermore, H&E staining of retinal sections confirmed engraftment of the grafts.

RPE grafts and immune cells were observed by immunohistochemical (IHC) analysis of retinal sections (f, g). As a result, proliferated graft cells (arrow) were observed in the section (DAPI staining only). In addition, inflammatory immune responses such as CD3, CD4, and MHC class II positive cells were not observed. A few Iba1-positive cells (microglia and macrophages) were observed around the transplanted area (choroid), but the transplanted RPE cells proliferated and engrafted. Therefore, transplanted allogeneic MHC class II KO iPS-RPE cells were engrafted without immune response.

### Example 5 Production of human HLA class II-deficient iPS cell-derived RPE cell, and immune response test

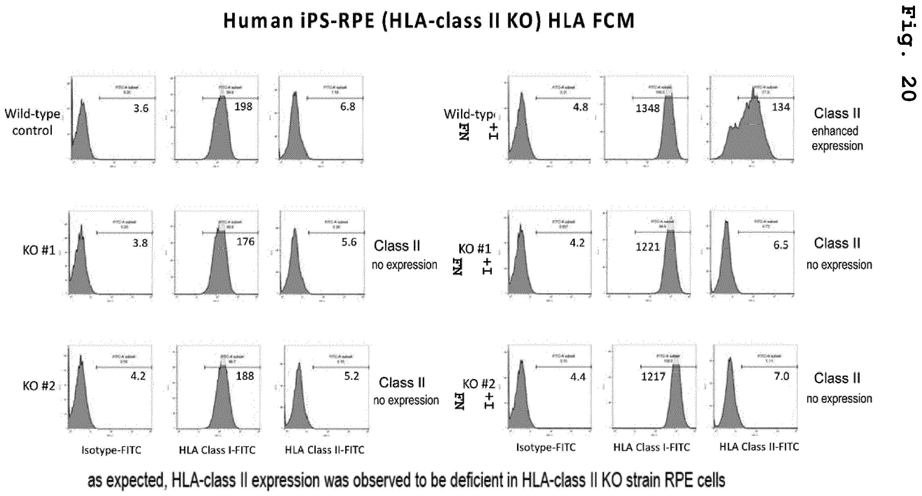

An MHC class II (HLA class II)-deficient strain was prepared from the iPS cell line QHJI01s04 according to the method described in Example 2, and differentiation thereof was induced to prepare RPE cells. HLA class II deficiency was confirmed by flow cytometry analysis (Fig. 20).

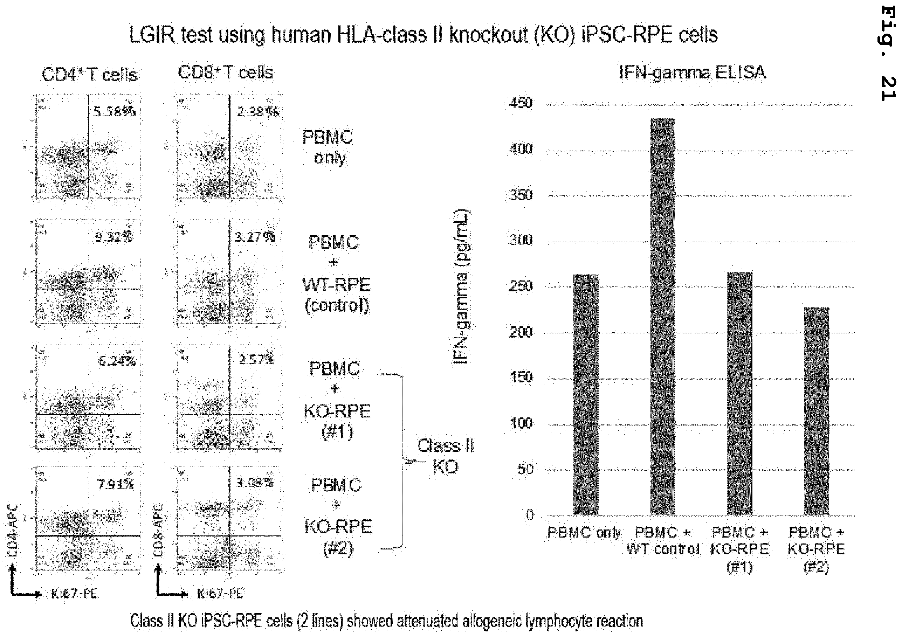

In the LGIR test, the WT control strain showed proliferation and activation of T cells, while the HLA class II-deficient iPS cell-derived RPE cells (QHJI01s04 strain: 2 lines) showed a weak T cell response (Fig. 21).

The above results suggest that HLA class II-deficient RPE cells have the effect of suppressing post-transplantation immune responses.

### [Industrial Applicability]

RPE cells derived from pluripotent stem cells in which class I was preserved and only class II was selectively destroyed can sufficiently suppress immune responses in allogeneic transplantation. Cells with minimal genetic modification can avoid the risk caused by failure of class I HLA protein to be presented on the cell surface that, when cells are infected with viruses, etc. or become tumors, antigens derived from the viruses or tumors cannot be presented, which may consequently assist the growth of the viruses and tumors.

Another advantage of preserving class I is that the attack of natural killer (NK) cell graft (RPE cell) can be avoided. Due to the expression of classical class I (HLA-ABC) and non-classical class I (HLA-E), the attack of NK cell on the graft is considered to be strikingly reduced.

This application is based on a US provisional patent application (No.63/158,661, filing date: March 9, 2021), the contents of which are incorporated in full herein.

## Claims

1. A method for producing a low immunogenic retinal pigment epithelial cell, comprising
step 1: a step of obtaining a pluripotent stem cell that does not express major histocompatibility complex (MHC) class II protein or lacks the function thereof, and
step 2: a step of inducing a retinal pigment epithelial cell from the pluripotent stem cell obtained in step 1.

2. The method according to claim 1, wherein the step 1 is performed by destroying or modifying an MHC class II-related gene.

3. The method according to claim 2, wherein the gene is destroyed or modified by genome editing.

4. The method according to claim 2, wherein the gene is destroyed or modified by a CRISPR-Cas9 method.

5. The method according to any one of claims 1 to 4, wherein the MHC class II is human leukocyte antigen (HLA) class II.

6. The method according to any one of claims 2 to 5, wherein the HLA class II-related gene is a gene encoding at least one selected from regulatory factor X-associated ankyrin-containing protein (RFXANK), regulatory factor v (RFX5), regulatory factor X-associated protein (RFXAP), class II transactivator (CIITA), HLA-DPA (α chain), HLA-DPB (β chain), HLA-DQA, HLA-DQB, HLA-DRA, HLA-DRB, HLA-DMA, HLA-DMB, HLA-DOA, and HLA-DOB.

7. The method according to claim 6, wherein the HLA class II-related gene is a gene encoding CIITA.

8. The method according to any one of claims 1 to 7, wherein the pluripotent stem cell is an embryonic stem cell (ES cell), an EG cell (Embryonic germ cell), or an induced pluripotent stem cell (iPS cell).

9. The method according to claim 8, wherein the pluripotent stem cell is derived from human.

10. A low immunogenic retinal pigment epithelial cell that does not express a major histocompatibility complex (MHC) class II protein or lacks the function thereof.

11. The cell according to claim 10, which is produced by the method according to any one of claims 1 to 9.

12. The cell according to claim 10 or 11, that expresses a major histocompatibility complex (MHC) class I protein, and does not lack the function thereof.

13. The cell according to any one of claims 10 to 12, wherein a major histocompatibility complex (MHC) class I-related gene is retained intact.

14. The cell according to claim 12 or 13, wherein the MHC class I is human leukocyte antigen (HLA) class I.

15. The cell according to claim 14, wherein the HLA class I is HLA-A, HLA-B, or HLA-C.

16. A pluripotent stem cell for inducing a low immunogenic retinal pigment epithelial cell that lacks a major histocompatibility complex (MHC) class II-related gene or lacks the function thereof.

17. The cell according to claim 16, wherein the MHC class II is human leukocyte antigen (HLA) class II.

18. The cell according to claim 17, wherein the HLA class II-related gene is a gene encoding at least one selected from regulatory factor X-associated ankyrin-containing protein (RFXANK), regulatory factor v (RFX5), regulatory factor X-associated protein (RFXAP), class II transactivator (CIITA), HLA-DPA (α chain), HLA-DPB (β chain), HLA-DQA, HLA-DQB, HLA-DRA, HLA-DRB, HLA-DMA, HLA-DMB, HLA-DOA, and HLA-DOB.

19. The cell according to claim 18, wherein the HLA class II-related gene is a gene encoding CIITA.

20. The cell according to any one of claims 16 to 19, wherein the pluripotent stem cell is an embryonic stem cell (ES cell), an EG cell (Embryonic germ cell), or an induced pluripotent stem cell (iPS cell).

21. The cell according to claim 20, which is derived from human.

22. A transplantation material comprising the retinal pigment epithelial cell according to any one of claims 10 to 15, or a retinal pigment epithelial cell derived from the pluripotent stem cell according to any one of claims 16 to 21.
